(19) Europäisches Patentamt
European Patent Office
Office européen des brevets

(11) EP 4 603 008 A1

(12) EUROPEAN PATENT APPLICATION
published in accordance with Art. 153(4) EPC

(43) Date of publication:
20.08.2025 Bulletin 2025/34

(51) International Patent Classification (IPC):
A61B 5/0245 (2006.01)    A61B 5/11 (2006.01)

(21) Application number: 23877269.3

(22) Date of filing: 10.10.2023

(52) Cooperative Patent Classification (CPC):
A61B 5/0245; A61B 5/11

(86) International application number:
PCT/JP2023/036666

(87) International publication number:
WO 2024/080265 (18.04.2024 Gazette 2024/16)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 11.10.2022  JP 2022163469

(71) Applicant: Minebea Mitsumi Inc.
Kitasaku-gun, Nagano 3890293 (JP)

(72) Inventors:
• HAYASHI, Yugo
Tama-shi, Tokyo 206-8567 (JP)

• MORISHITA, Eizaburo
Tama-shi, Tokyo 206-8567 (JP)
• WADA, Satoru
Tama-shi, Tokyo 206-8567 (JP)
• SHIMIZU, Nobutaka
Tama-shi, Tokyo 206-8567 (JP)
• HATTA, Kunihiko
Kitasaku-gun, Nagano 389-0293 (JP)
• NISHIKATA, Nobutaka
Kitasaku-gun, Nagano 389-0293 (JP)

(74) Representative: Winter, Brandl - Partnerschaft mbB
Alois-Steinecker-Straße 22
85354 Freising (DE)

(54) **HEART RATE ACQUISITION DEVICE AND BED SYSTEM**

(57) A heart rate acquisition device (30) for acquiring a heart rate of a subject (S) on a bed (BD) includes a heartbeat waveform acquisition unit (31) for acquiring heartbeat waveforms indicating temporal fluctuations of load values corresponding to a heartbeat of the subject based on outputs of load detectors (11, 12, 13, 14) located at the bed, a cardiac cycle estimation unit (33) for obtaining an estimated value of a cardiac cycle of the subject based on transitions of the heartbeat waveform, and a heart rate calculation unit (34) for calculating a heart rate of the subject based on the heartbeat waveform and the estimated value.

FIG. 1

EP 4 603 008 A1

**Description**

Technical Field

The present invention relates to a heart rate acquisition device and a bed system.

Background Art

In the medical and long-term care fields, detecting the load of a subject on a bed via a load detector and acquiring biological information, such as respiratory rate and heart rate, of the subject based on the detected load has been proposed.

Patent Document 1 discloses calculating an autocorrelation function of a heartbeat waveform acquired based on an output of a load detector, and calculating a heart rate of a subject based on a peak of the autocorrelation function.

Citation List

Patent Literature

Patent Document 1: JP 2022-72436 A

Summary of Invention

Technical Problem

In the acquisition of biological information based on load detection, higher accuracy, higher efficiency, and the like are required.

One object of the present invention is to provide a heart rate acquisition device and a bed system capable of acquiring a heart rate with higher accuracy.

Another object of the present invention is to provide a heart rate acquisition device and a bed system capable of acquiring a heart rate more efficiently.

A yet another object of the present invention is to provide a heart rate acquisition device and a bed system capable of more appropriately displaying the acquired heart rate.

Solution to Problem

According to a first aspect of the present invention, there is provided a heart rate acquisition device configured to acquire a heart rate of a subject on a bed, the heart rate acquisition device including a heartbeat waveform acquisition unit configured to acquire a heartbeat waveform indicating a temporal fluctuation of a load value corresponding to a heartbeat of the subject based on an output of a load detector located at the bed, a cardiac cycle estimation unit configured to obtain an estimated value of a cardiac cycle of the subject based on transition of the heartbeat waveform, and a heart rate calculation unit configured to calculate a heart rate of the subject based on the heartbeat waveform and the estimated value.

According to a second aspect of the present invention, there is provided a heart rate acquisition device configured to acquire a heart rate of a subject on a bed, the heart rate acquisition device including a heart rate calculation unit configured to calculate a heart rate of the subject based on a temporal fluctuation of an output of a load detector located at the bed corresponding to a heartbeat of the subject, and a display unit configured to display the heart rate calculated by the heart rate calculation unit, and a display control unit configured to control display content of the display unit. The display control unit changes a display mode of the heart rate calculated by the heart rate calculation unit at the display unit between a period when the subject is present on the bed and a period when the subject is not present on the bed.

According to a third aspect of the present invention, there is provided a heart rate acquisition device configured to acquire a heart rate of a subject on a bed, the heart rate acquisition device including a heartbeat waveform acquisition unit configured to acquire a heartbeat waveform indicating a temporal fluctuation of a load value corresponding to a heartbeat of the subject based on an output of a load detector located at the bed, and a heart rate calculation unit configured to calculate a heart rate of the subject based on the heartbeat waveform. Calculation of the heart rate at the heart rate calculation unit includes: calculating an autocorrelation function of the heartbeat waveform, detecting a peak of the autocorrelation function, and calculating the heart rate based on a position of the peak detected, and calculation of the autocorrelation function in the heart rate calculation unit includes: calculating a first autocorrelation function of the heartbeat waveform included in a first period, calculating a second autocorrelation function of the heartbeat waveform included in a second period after the first period, calculating a third autocorrelation function of the heartbeat waveform included in a third period after the second period, calculating a sum of the first autocorrelation function and the second autocorrelation function at a first time as the autocorrelation function, and calculating a sum of the second autocorrelation function and the third autocorrelation function at a second time after the first time as the autocorrelation function.

According to a fourth aspect of the present invention, there is provided a bed system including a bed, a load detector located at the bed, and the heart rate acquisition device according to the first aspect, the second aspect, or the third aspect.

Advantageous Effects of Invention

The heart rate acquisition device and the bed system according to the present invention can acquire a heart rate with higher accuracy.

The heart rate acquisition device and the bed system according to the present invention can acquire a heart rate more efficiently.

The heart rate acquisition device and the bed system according to the present invention can display the acquired heart rate more appropriately.

Brief Description of Drawings

FIG. 1 is a block diagram illustrating a configuration of a biological information acquisition device according to an embodiment of the present invention.

FIG. 2 is an explanatory diagram illustrating an arrangement of load detectors with respect to a bed.

FIG. 3 is a flowchart illustrating steps of acquiring a heart rate of a subject using the biological information acquisition device.

FIG. 4(a), FIG. 4(b), FIG. 4(c), and FIG. 4(d) are graphs illustrating examples of heartbeat waveforms based on outputs from the four load detectors placed under legs of the bed, respectively.

FIG. 5 is a flowchart illustrating details of a cardiac cycle estimation step.

FIG. 6 is a graph illustrating an example of a heartbeat waveform together with bottoms, rising zero crossing points, peaks, and falling zero crossing points of the heartbeat waveform.

FIG. 7 is a table showing tracking of a heartbeat waveform in the cardiac cycle estimation step, and mainly shows a relationship between states of the heartbeat waveform and state transition variables.

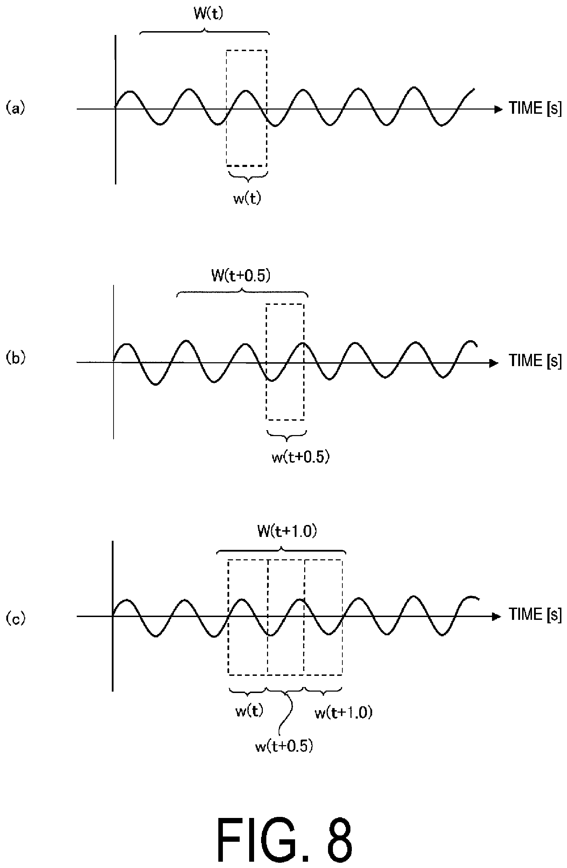

FIG. 8(a), FIG. 8(b), and FIG. 8(c) are explanatory diagrams for explaining significance of a heart rate calculation unit dividing an autocorrelation function of the heartbeat waveform before calculation. FIG. 8(a) is a graph for explaining how part of the autocorrelation function of the heartbeat waveform is calculated at time t. FIG. 8(b) is a graph for explaining how part of the autocorrelation function of the heartbeat waveform is calculated at time t + 0.5. FIG. 8(c) is a graph for explaining how part of the autocorrelation function of the heartbeat waveform is calculated at time t + 1.0.

FIG. 9 is a flowchart illustrating details of a heart rate calculation step.

FIG. 10(a) to FIG. 10(h) are explanatory diagrams for explaining a procedure for calculating the autocorrelation function in the heart rate calculation unit. FIG. 10(a) illustrates an example of a heartbeat waveform together with sampled values of the heartbeat waveform. FIG. 10(b) to FIG. 10(h) are explanatory diagrams illustrating contents of sampled values stored at arrays located at a specified area of a storage unit in a calculation step of the autocorrelation function.

FIG. 11(a) and FIG. 11(b) are explanatory diagrams for explaining an interpolation performed in the heart rate calculation step. FIG. 11(a) is a graph illustrating data points around a peak of the autocorrelation function detected by peak detection. FIG. 11(b) is a graph illustrating a quadratic curve calculated based on the data points of FIG. 11(a) and a peak of the quadratic curve.

FIG. 12(a) and FIG. 12(b) illustrate modes of display of a respiratory rate and a heart rate at the display unit, respectively. FIG. 12(a) illustrates a state with the respiratory rate and the heart rate displayed at the display unit. FIG. 12(b) illustrates a state with the respiratory rate and the heart rate not displayed at the display unit.

Description of Embodiments

Embodiments

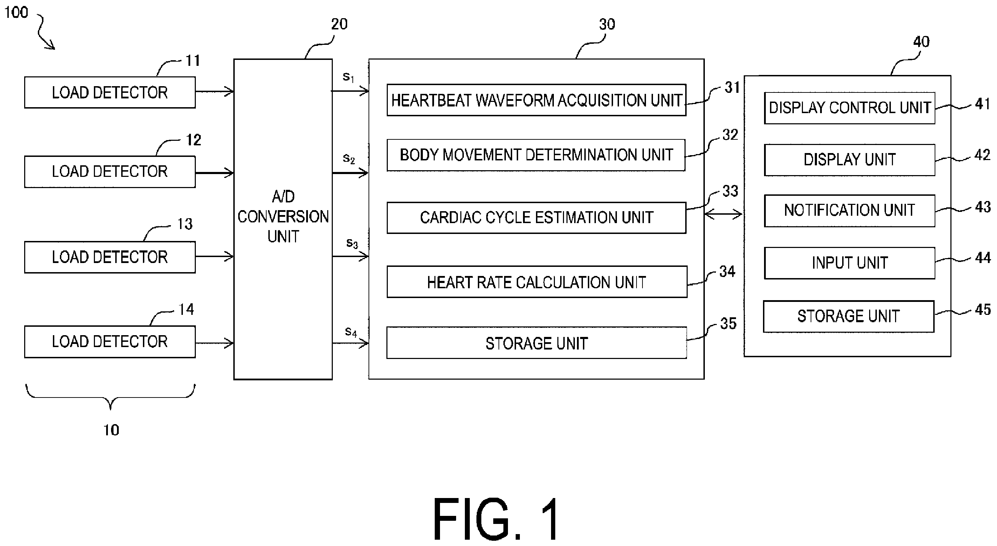

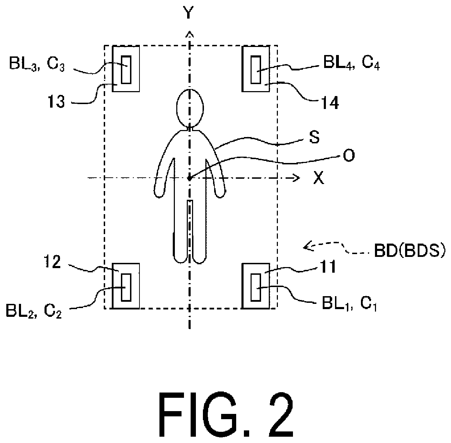

A biological information acquisition system 100 (FIG. 1) according to an embodiment of the present invention will be described with an example. In the example, this system is used together with a bed BD (FIG. 2) to calculate (estimate) a heart rate of a subject S on the bed BD.

Configuration of Biological Information Acquisition System 100

As illustrated in FIG. 1, the biological information acquisition system 100 according to the present embodiment primarily includes a load detection unit 10, a biological information acquisition unit (heart rate acquisition device) 30, and a user terminal 40. The load detection unit 10 and the biological information acquisition unit 30 are connected via an A/D conversion unit 20. The biological information acquisition unit 30 and the user terminal 40 are connected by wiring or wirelessly.

The load detection unit 10 includes four load detectors 11, 12, 13, and 14. Each of the load detectors 11, 12, 13, and 14 is a load detector for detecting a load by using, for example, a beam type load cell. The load detectors 11, 12, 13, and 14 are each connected to the A/D conversion unit 20 by wiring or wirelessly.

As illustrated in FIG. 2, the four load detectors 11 to 14 are located under casters $C_1$, $C_2$, $C_3$, and $C_4$ attached to lower end parts of legs $BL_1$, $BL_2$, $BL_3$, and $BL_4$ at four corners of the bed BD used by the subject S, respectively.

The A/D conversion unit 20 is separately connected to the load detection unit 10 and the biological information acquisition unit 30 by wiring or wirelessly. The A/D conversion unit 20 includes an A/D converter for converting an analog signal from the load detection unit 10 into a digital signal.

The biological information acquisition unit 30 mainly includes a heartbeat waveform acquisition unit 31, a body movement determination unit 32, a cardiac cycle estimation unit 33, a heart rate calculation unit 34, and a storage unit 35. The heartbeat waveform acquisition unit 31, the body movement determination unit 32, the cardiac cycle estimation unit 33, and the heart rate calculation unit 34 are, as an example, constructed inside a control unit (controller) (not illustrated) included in the biological information acquisition unit 30. Operations of the heartbeat waveform acquisition unit 31, the body movement determination unit 32, the cardiac cycle estimation unit 33, and the heart rate calculation unit 34 will be described later.

The storage unit 35 is a storage device for storing data used in the biological information acquisition unit 30. The storage unit 35 may be a hard disk, a nonvolatile memory, or the like, as an example.

The user terminal 40 includes a display control unit 41, a display unit 42, a notification unit 43, an input unit 44, and a storage unit 45.

The display control unit 41 is constructed inside a control unit (controller) (not illustrated) included in the user terminal 40, as an example. The display control unit 41 acquires various types of information, such as the heart rate, acquired by the biological information acquisition unit 30 from the biological information acquisition unit 30, and displays the various types of information at the display unit 42 (details will be described later).

The display unit 42 visually displays the information, such as the heart rate, acquired from the biological information acquisition unit 30 to the user of the biological information acquisition system 100. The display unit 42 may be, for example, a monitor such as a liquid crystal monitor.

The notification unit 43 aurally gives a specified notification based on the information, such as the heart rate, acquired from the biological information acquisition unit 30. The notification unit 43 may be, for example, a speaker. The display control unit 41 may control operation of the notification unit 43.

The input unit 44 is an interface for providing input to the biological information acquisition system 100. The input unit 44 may be, for example, a keyboard and a mouse.

The storage unit 45 is a storage device for storing data used at the user terminal 40. The storage unit 45 may be a hard disk, a nonvolatile memory, or the like, as an example.

Operation of Biological Information Acquisition System 100

Operation of acquiring a heart rate of a subject on a bed using the biological information acquisition system 100 having such a configuration will be described.

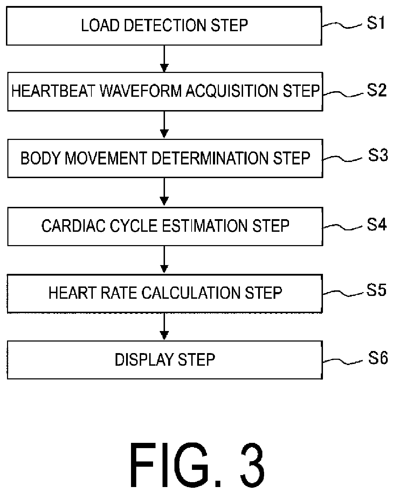

Acquiring the heart rate of the subject S using the biological information acquisition system 100 includes a load detection step S1, a heartbeat waveform acquisition step S2, a body movement determination step S3, a cardiac cycle estimation step S4, a heart rate calculation step S5, and a display step S6, as illustrated in a flowchart in FIG. 3.

In outline, in the load detection step S1, a load of the subject S is detected using the load detectors 11 to 14. In the heartbeat waveform acquisition step S2, heartbeat waveforms are extracted by filtering from temporal fluctuations of values detected by the load detectors 11 to 14. In the body movement determination step S3, presence or absence of body movement of the subject S is determined based on an amplitude of the heartbeat waveform. In the cardiac cycle estimation step S4, a cardiac cycle of the subject S is estimated based on the heartbeat waveform. In the heart rate calculation step S5, the heart rate of the subject S is calculated based on the heartbeat waveform, the presence or absence of body movement, and the estimated value of the cardiac cycle. In the display step S6, the calculated heart rate is displayed at the display unit 42.

Load Detection Step S1

In the load detection step S1, the load detectors 11, 12, 13, and 14 are used to detect the load of the subject S on the bed BD. The load of the subject S on the bed BD is applied separately to the load detectors 11 to 14 located under the legs $BL_1$ to $BL_4$ at the four corners of the bed BD and is detected separately by these load detectors.

The load detectors 11 to 14 each detect loads (load changes) and output the loads to the A/D conversion unit 20 as analog signals. The A/D conversion unit 20 converts the analog signals into digital signals with a sampling period of, for example, 5 milliseconds (0.005 seconds), and outputs the digital signals (hereinafter, "load signals") to the biological information acquisition unit 30. Hereinafter, the load signals acquired by digitally converting the analog signals output from the load detectors 11, 12, 13, and 14 at the A/D conversion unit 20 are referred to as load signals $s_1$, $s_2$, $s_3$, and $s_4$, respectively.

Heartbeat Waveform Acquisition Step S2

In the heartbeat waveform acquisition step S2, the heartbeat waveform acquisition unit 31 acquires a heartbeat waveform of the subject S from each of the load signals $s_1$ to $s_4$.

In the present specification and the present invention, "heartbeat waveform" means a waveform indicating a temporal fluctuation of a load value corresponding to the heartbeat of a subject. One period of the heartbeat waveform corresponds to one period of the heartbeat. The amplitude of the heartbeat waveform is correlated with the amount of blood flowing with a single heartbeat. If the other conditions are the same, a larger amount of blood flowing due to the heart beating leads to a heartbeat waveform with a larger amplitude.

To be specific, the heartbeat waveform acquisition unit 31 acquires a heartbeat waveform, for example, by the following method.

Since the human heart beats about 40 to 150 times per minute, the frequency of the human heartbeat is about 0.66 to 2.5 Hz (hereinafter referred to as a "heartbeat band"). Thus, the heartbeat waveform acquisition unit 31 extracts components having frequencies at the heartbeat band from the load signals $s_1$ to $s_4$, respectively, using a bandpass filter, and the extracted components are heartbeat waveforms HW1 to HW4.

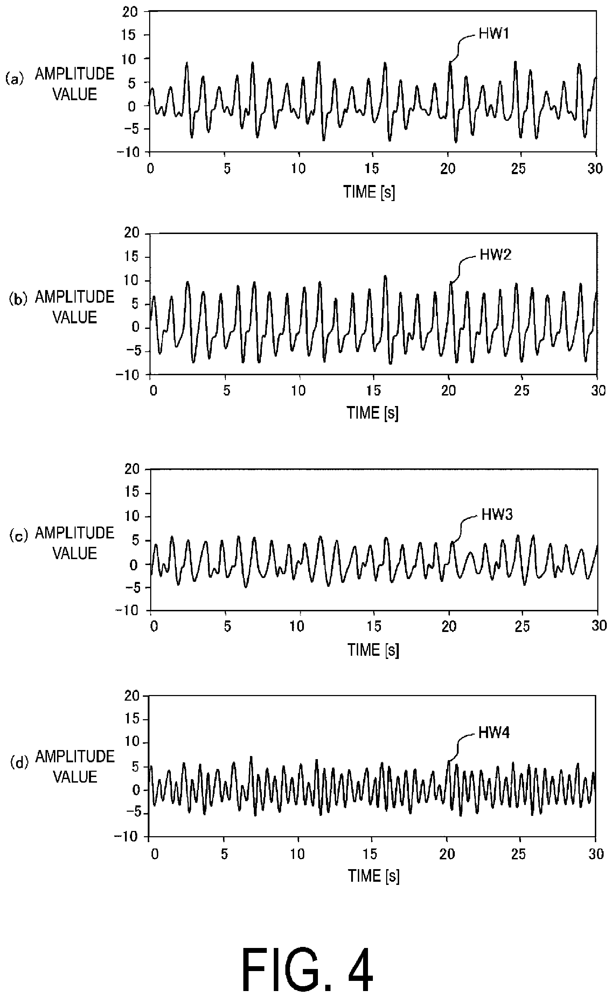

Examples of the heartbeat waveform HW1 extracted from the load signal $s_1$, the heartbeat waveform HW2 extracted from the load signal $s_2$, the heartbeat waveform HW3 extracted from the load signal $s_3$, and the heartbeat waveform HW4 extracted from the load signal $s_4$ are shown in FIG. 4(a), FIG. 4(b), FIG. 4(c), and FIG. 4(d), respectively. The heartbeat waveforms HW1 to HW4 shown in FIG. 4(a) to FIG. 4(d) are heartbeat waveforms of the subject S during the same period (time 0 s to time 30 s) as each other.

In addition, the heartbeat waveform acquisition unit 31 performs downsampling on each of the acquired heartbeat waveforms HW1 to HW4 in order to reduce processing load. In the present embodiment, sampling frequency conversion is performed with a sampling period of 5 milliseconds (0.005 seconds) to 10 milliseconds (0.01 seconds). Note that downsampling does not have to be performed.

Body Movement Determination Step S3

In the body movement determination step S3, the body movement determination unit 32 determines whether the body movement has occurred at the subject S based on a comparison between amplitudes AM of the heartbeat waveforms HW1 to HW4 and thresholds.

Here, in the present invention and the present specification, "body movement" means movement of the head, torso (trunk), and limbs of the subject. The body movement does not include the movement of organs, blood vessels, and the like associated with respiration, heartbeat, and the like. As an example, the body movement can be classified into large body movement involving movement of the torso (trunk) of the subject and small body movement involving only movement of the limbs and the head of the subject. An example of the large body movement is turning over, getting up, or the like, and an example of the small body movement is movement of the limbs, head, or the like during sleep.

The body movement determination unit 32 determines the presence or absence of body movement based on a comparison between the amplitudes AW of the heartbeat waveforms HW1 to HW4 and the thresholds. To be specific, for example, the body movement determination unit 32 determines that the body movement occurs at the subject S when the amplitude AW of at least one of the heartbeat waveforms HW1 to HW4 is greater than a positive-side body movement threshold THp or smaller than a negative-side body movement threshold THn.

The body movement thresholds THp and THn can be set as desired. To be specific, for example, taking into consideration magnitudes of the amplitudes AM of the heartbeat waveforms HW1 to HW4 in the past, appropriate values can be set for determining that the body movement occurs at the subject S when the amplitudes AM (positive values) are greater than the positive thresholds, and appropriate values can be set for determining that the body movement occurs at the subject S when the amplitudes AM (negative values) are smaller than the negative thresholds. By using only positive thresholds, it may be determined that body movement occurs at the subject S when absolute values of the amplitudes AM are greater than these thresholds.

The body movement determination unit 32 sets a body movement occurrence flag during a period when the body movement determination unit 32 determines that body movement occurs at the subject S. The body movement occurrence flag is set, for example, at a specified area secured at the storage unit 35.

Cardiac Cycle Estimation Step S4

In the cardiac cycle estimation step S4, the cardiac cycle estimation unit 33 estimates the cardiac cycle of the subject S based on the heartbeat waveforms HW1 to HW4.

As described above, one period of the heartbeat waveform corresponds to one period of the heartbeat. Therefore, the cardiac cycle estimation unit 33 estimates the cardiac cycle of the subject S by tracking transition of at least one of the heartbeat waveform HW1 to the heartbeat waveform HW4 and determining a gap (length) of one period of the heartbeat waveform.

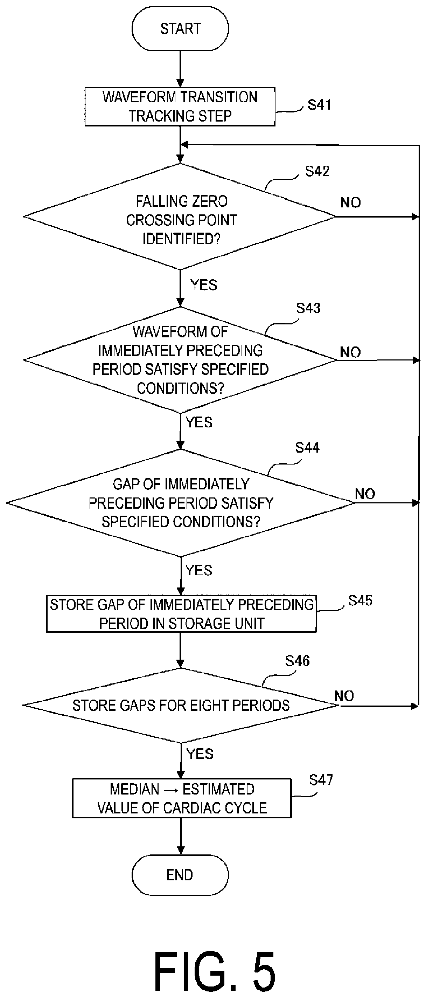

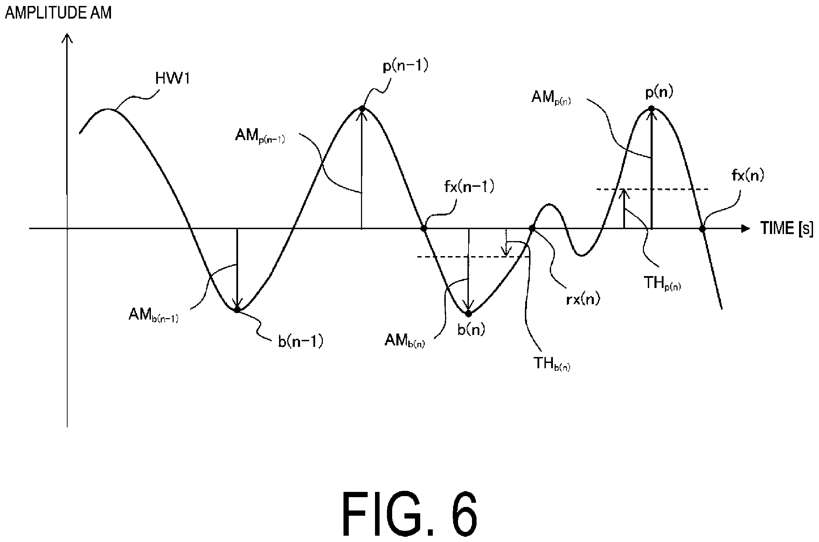

To be specific, the cardiac cycle estimation unit 33 estimates the cardiac cycle of the subject S, for example, according to a procedure illustrated in a flowchart in FIG. 5. Here, an example of estimating the cardiac cycle of the subject S by tracking the transition of the heartbeat waveform HW1 (FIG. 6) is described.

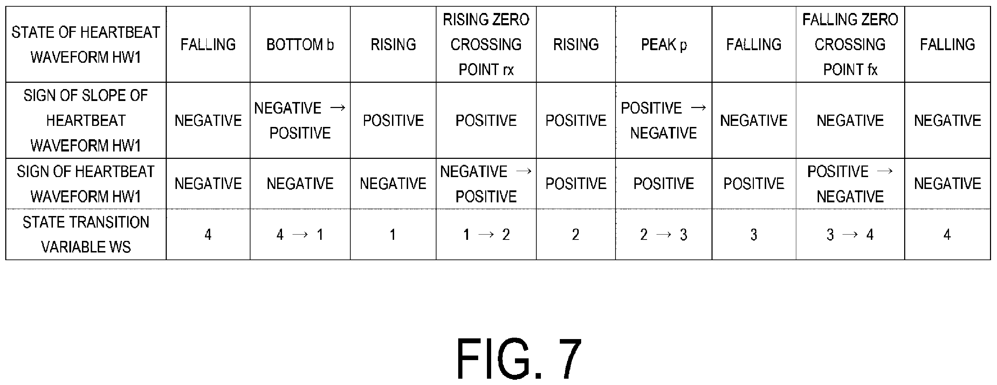

In the cardiac cycle estimation step S4, the cardiac cycle estimation unit 33 constantly executes a waveform transition tracking step S41. In the waveform transition tracking step S41, the cardiac cycle estimation unit 33 tracks the transition of the heartbeat waveform HW1 based on a sign of a slope of the heartbeat waveform HW1, a sign of the heartbeat waveform HW1, and a state transition variable WS (FIG. 7).

To be specific, the cardiac cycle estimation unit 33 sequentially identifies a bottom b of the heartbeat waveform HW1, a zero crossing point rx at a rising edge of the heartbeat waveform HW1 (hereinafter referred to as a "rising zero crossing point rx"), a peak p of the heartbeat waveform HW1, and a zero crossing point fx at a falling edge of the heartbeat waveform HW1 (hereinafter referred to as a "falling zero crossing point fx").

Here, the state transition variable WS is a variable indicating a state of the heartbeat waveform HW1. When the state transition variable WS is "1", the heartbeat waveform HW1 is in a state from the bottom b toward the rising zero crossing point rx. When the state transition variable WS is "2", the heartbeat waveform HW1 is in a state from the rising zero crossing point rx toward the peak p. When the state transition variable WS is "3", the heartbeat waveform HW1 is in a state from the peak p toward the falling zero crossing point fx. When the state transition variable WS is "4", the heartbeat waveform HW1 is in a state from the falling zero crossing point fx to the bottom b.

To be specific, the cardiac cycle estimation unit 33 identifies the bottom b, the rising zero crossing point rx, the peak p, and the falling zero crossing point fx, for example, as follows. Here, an example of identifying a bottom b(n) being an nth bottom b, a rising zero crossing point rx(n) being an nth rising zero crossing point, a peak p(n) being an nth peak p, and a falling zero crossing point fx(n) being an nth falling zero crossing point fx will be described.

(1) Identification of Bottom b

As illustrated in FIG. 6 and FIG. 7, when the state transition variable WS is "4" and the sign of the slope of the heartbeat waveform HW1 changes from negative to positive at a certain point in time, and the amplitude AM of the heartbeat waveform HW1 at the certain point in time is smaller than a threshold $TH_{b(n)}$ (bottom amplitude threshold), the cardiac cycle estimation unit 33 identifies that the bottom b(n) of the heartbeat waveform HW1 exists at the certain point in time. As an example, the threshold $TH_{b(n)}$ may be one third of an amplitude $AM_{b(n-1)}$ of the respiratory waveform HW1 at a bottom b(n-1) identified one period earlier.

When the cardiac cycle estimation unit 33 identifies the bottom b(n), the cardiac cycle estimation unit 33 stores an amplitude $AM_{b(n)}$ of the heartbeat waveform HW1 at the bottom b(n) at the storage unit 35. Further, the cardiac cycle estimation unit 33 shifts the state transition variable WS from "4" to "1". When the state transition variable WS is "1", the cardiac cycle estimation unit 33 waits for arrival of the rising zero crossing point rx.

(2) Identification of Rising Zero Crossing Point rx

When the state transition variable WS is "1" and the sign of the heartbeat waveform HW1 changes from negative to positive at a certain point in time, the cardiac cycle estimation unit 33 identifies that the rising zero crossing point rx(n) exists at the certain point in time.

When the cardiac cycle estimation unit 33 identifies the rising zero crossing point rx(n), the cardiac cycle estimation unit 33 shifts the state transition variable WS from "1" to "2". When the state transition variable WS is "2", the cardiac cycle estimation unit 33 waits for arrival of the peak p.

(3) Identification of Peak p

When the state transition variable WS is "2" and the sign of the slope of the heartbeat waveform HW1 changes from positive to negative at a certain point in time, and the amplitude AM of the heartbeat waveform HW1 at the certain point in time is greater than a threshold $TH_{p(n)}$ (peak amplitude threshold), the cardiac cycle estimation unit 33 identifies that the peak p(n) of the heartbeat waveform HW1 exists at the certain point in time. As an example, the threshold $TH_{p(n)}$ may be one third of an amplitude $AM_{p(n-1)}$ of the respiratory waveform HW1 at a peak p(n-1) identified one period earlier.

When the cardiac cycle estimation unit 33 identifies the peak p(n), the cardiac cycle estimation unit 33 stores an amplitude $AM_{p(n)}$ of the heartbeat waveform HW1 at the peak p(n) at the storage unit 35. Further, the cardiac cycle estimation unit 33 shifts the state transition variable WS from "2" to "3". When the state transition variable WS is "3", the cardiac cycle estimation unit 33 waits for arrival of the falling zero crossing point fx.

(4) Identification of Falling Zero Crossing Point fx

When the state transition variable WS is "3" and the sign of the heartbeat waveform HW1 changes from positive to negative at a certain point in time, the cardiac cycle estimation unit 33 identifies that the falling zero crossing point fx(n) exists at the certain point in time.

When the cardiac cycle estimation unit 33 identifies the falling zero crossing point fx(n), the cardiac cycle estimation unit 33 shifts the state transition variable WS from "3" to "4". When the state transition variable WS is "4", the cardiac cycle estimation unit 33 waits for arrival of the bottom b.

The cardiac cycle estimation unit 33 executes step S42 in parallel with the waveform transition tracking step S41 and determines whether the falling zero crossing point fx has been identified in the waveform transition tracking step S41.

If the cardiac cycle estimation unit 33 determines that the falling zero crossing point fx has not been identified (S42: NO), the cardiac cycle estimation unit 33 executes step S42 again. If the cardiac cycle estimation unit 33 determines that the falling zero crossing point fx has been identified (S42: YES), the cardiac cycle estimation unit 33 proceeds to step S43.

In step S43, the cardiac cycle estimation unit 33 determines whether the heartbeat waveform HW1 satisfies specified conditions in a period immediately preceding the identified zero crossing point fx.

To be specific, the cardiac cycle estimation unit 33 determines whether the amplitude AM (positive value) at the peak p identified in the immediately preceding period is smaller than a body movement threshold THp and whether the amplitude AM (negative value) at the bottom b identified in the immediately preceding period is greater than a body movement threshold THn. The peak p and the bottom b identified in the immediately preceding period are, for example, p(n) and b(n) at the timing when the falling zero crossing point fx(n) is identified. The body movement thresholds THp and THn may be the same values as the body movement thresholds THp and THn used in the body movement determination step S3.

Further, the cardiac cycle estimation unit 33 determines whether the gap PR of the immediately preceding period is within a specified range. The gap PR of the immediately preceding period is, for example, a length (time) from a falling zero crossing point fx(n-1) to the falling zero crossing point fx(n) at the timing when the falling zero crossing point fx(n) is identified.

If the amplitude AM (positive value) at the peak p identified in the immediately preceding period is greater than the body movement threshold THp, and/or if the amplitude AM (negative value) at the bottom b identified in the immediately preceding period is smaller than the body movement threshold THn, or if the gap PR of the immediately preceding period is not within the specified range (S43: NO), the cardiac cycle estimation unit 33 returns to step S42.

If the amplitude AM (positive value) at the peak p identified in the immediately preceding period is equal to or less than the body movement threshold THp, the amplitude AM (negative value) at the bottom b identified in the immediately preceding period is equal to or more than the body movement threshold THn, and the gap PR of the immediately preceding period is within the specified range (S43: YES), the cardiac cycle estimation unit 33 proceeds to step S44. To be specific, for example, when the gap PR of the immediately preceding period is equal to or greater than a specified value, the cardiac cycle estimation unit 33 determines that the PR is within the specified range.

In step S44, the cardiac cycle estimation unit 33 determines whether a fluctuation ratio of the gap PR of the immediately preceding period to the gap PR of the period one period before this immediately preceding period is within a specified range. Note that when the gap PR of the immediately preceding period is a length (time) from the falling zero crossing point fx(n-1) to the falling zero crossing point fx(n), the gap PR of the period one period before this immediately preceding period is a length (time) from a falling zero crossing point fx(n-2) to the falling zero crossing point fx(n-1).

To be specific, for example, when the fluctuation ratio of the gap PR of the immediately preceding period to the gap PR of one period before this immediately preceding period is 50% or more and 200% or less (i.e., when the gap PR of the immediately preceding period is 1/2 or more and twice or less than the gap PR of the period one period before this immediately preceding period), the cardiac cycle estimation unit 33 determines that the fluctuation ratio of the gap PR of the immediately preceding period to the gap PR of the period one period before this immediately preceding period is within the specified range.

If the cardiac cycle estimation unit 33 determines that the fluctuation ratio of the gap PR of the immediately preceding period to the gap PR of the period one period before this immediately preceding period is not within the specified range (S44: NO), the cardiac cycle estimation unit 33 returns to step S42. If the cardiac cycle estimation unit 33 determines that the fluctuation ratio of the gap PR of the immediately preceding period to the gap PR of the period one period before this immediately preceding period is within the specified range (S44: YES), the cardiac cycle estimation unit 33 proceeds to step S45.

In step S45, the cardiac cycle estimation unit 33 stores the gap PR of the immediately preceding period at a specified area of the storage unit 35. At this time, when the gaps PR for eight periods have already been stored at this area, the gap PR for the oldest period is deleted. That is, the storage to the storage unit 35 is done by a FIFO method.

In step S46, the cardiac cycle estimation unit 33 determines whether the gaps PR for the eight periods are stored at the specified area of the storage unit 35. If the gaps RP for the eight periods are stored at the specified area of the storage unit 35 (S46: YES), the cardiac cycle estimation unit 33 outputs a median of the PR for the eight periods to the heart rate calculation unit 34 as an estimated value of the cardiac cycle of the subject S (step S47). If the periods RP for the eight periods are not stored at the specified area of the storage unit 35 (S46: NO), the cardiac cycle estimation unit 33 returns to step S42.

Heart Rate Calculation Step S5

In the heart rate calculation step S5, the heart rate calculation unit 34 calculates (estimates) the heart rate of the subject S based on the states of temporal fluctuations of the heartbeat waveforms HW1 to HW4, the estimated value of the cardiac cycle estimated in the cardiac cycle estimation step S4, and the presence or absence of body movement of the subject S.

The heart rate calculation unit 34 calculates the heart rate of the subject S based on the following principle, in outline.

First, the heart rate calculation unit 34 calculates an autocorrelation function (autocorrelation coefficient) in the time domain for each of the heartbeat waveforms HW1 to HW4.

The calculated autocorrelation function indicates degree of coincidence between the heartbeat waveforms HW1 to HW4 each and waveforms obtained by shifting these heartbeat waveforms by a specified lag time in a time axis direction, using the lag time as a variable. The degree of coincidence between a periodic waveform and a waveform obtained by shifting this periodic waveform by a lag time in the time axis direction is the highest when the lag time is equal to the period of the waveform. Therefore, the value of the lag time when the autocorrelation function shows a peak indicates the period of each of the heartbeat waveforms HW1 to HW4.

In view of the above, the heart rate calculation unit 34 detects the peak of the autocorrelation function for each of the heartbeat waveforms HW1 to HW4 and determines the lag time corresponding to the detected peak to be the period of the heartbeat waveform. Then, the heart rate calculation unit 34 calculates an estimated value of the heart rate of the subject S based on the determined period.

Note that in the present embodiment, the heart rate calculation unit 34 calculates the autocorrelation function using a calculation window of 1.65 seconds. That is, the heart rate calculation unit 34 calculates the autocorrelation function with a lag time in a range of 0 seconds to 1.65 seconds. A human heart rate is usually at least about 40 [beats/minute], and a period of a heartbeat waveform is at most about 1.5 seconds. Therefore, by calculating the autocorrelation function using a calculation window of 1.65 seconds, an increase in an amount of calculation processing can be suppressed while accurately determining the period of the heartbeat waveform.

In addition, in the present embodiment, the heart rate calculation unit 34 divides the autocorrelation function before calculation, thereby suppressing an increase in the amount of calculation processing. This concept will be described with reference to FIG. 8(a) to FIG. 8(c).

In a state shown in FIG. 8(a), an autocorrelation function ACF(t) between a waveform w(t) for the past 0.5 seconds from time t and a waveform W(t) for the past 1.65 seconds from time t is calculated. In addition, in a state shown in FIG. 8(b), an autocorrelation function ACF(t + 0.5) between a waveform w(t + 0.5) for the past 0.5 seconds from time t + 0.5 and a waveform W(t + 0.5) for the past 1.65 seconds from time t + 0.5 is calculated. Further, in a state shown in FIG. 8(c), an autocorrelation function ACF(t + 1.0) between a waveform w(t + 1.0) for the past 0.5 seconds from time t + 1.0 and a waveform W(t + 1.0) for the past 1.65 seconds from time t + 1.0 is calculated.

Here, in the state shown in FIG. 8(c), calculation of an autocorrelation function between a waveform for the past 1.5 seconds from time t + 1.0 and the waveform W(t + 1.0) for the past 1.65 seconds from time t + 1.0 will be considered. In this case, as can be seen from FIG. 8(a) to FIG. 8(c), the calculated autocorrelation function is equal to a sum of the autocorrelation function ACF(t), the autocorrelation function ACF(t + 0.5), and the autocorrelation function ACF(t + 1.0).

That is, in the state shown in FIG. 8(c), when the autocorrelation function of the waveform for the past 1.5 seconds from time t + 1.0 is calculated using the calculation window of 1.65 seconds, the amount of calculation required to calculate the autocorrelation function of the waveform for the past 1.5 seconds from time t + 1.0 and the waveform W(t + 1.0) for the past 1.65 seconds from time t + 1.0 is large. On the other hand, the amount of calculation can be reduced by calculating the autocorrelation function ACF(t + 1.0) between the waveform w(t + 1.0) for the past 0.5 seconds from time t + 1.0 and the waveform W(t + 1.0) for the past 1.65 seconds from time t + 1.0 and adding the autocorrelation function ACF(t + 1.0) to the autocorrelation function ACF(t) and the autocorrelation function ACF(t + 0.5). The autocorrelation function ACF(t) and the autocorrelation function ACF(t + 0.5) have already been calculated.

Thus, at each timing when the autocorrelation function is calculated, the cardiac cycle calculation unit 34 according to the present embodiment does not calculate the autocorrelation function for the entire target waveform at each timing, but calculates the autocorrelation function for a most recent part of the target waveform at each timing, and adds the calculated autocorrelation function to the autocorrelation function calculated at the past timing, thereby suppressing an increase in the amount of calculation processing.

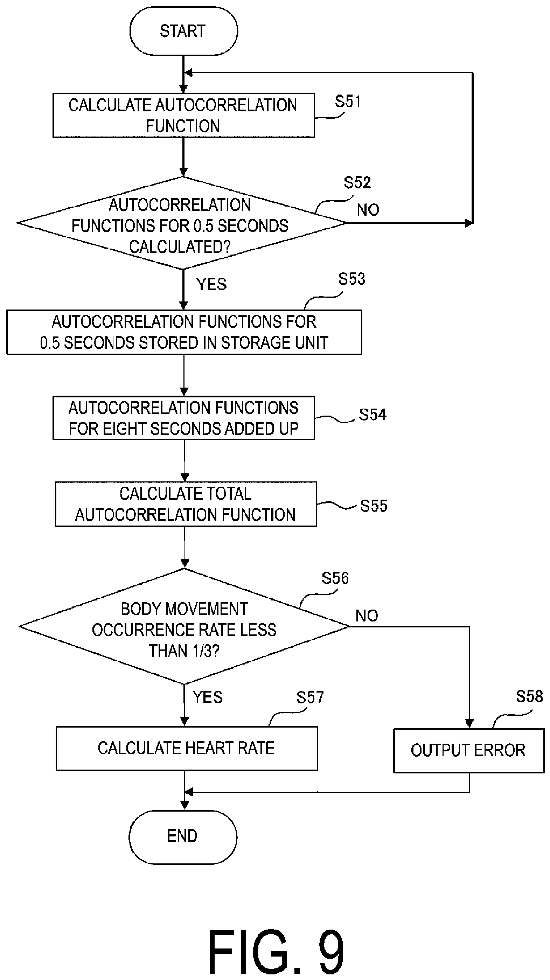

To be specific, for example, the heart rate calculation unit 34 calculates the heart rate of the subject S in the following procedure, according to a flowchart illustrated in FIG. 9.

In step S51, the heart rate calculation unit 34 sequentially calculates the autocorrelation function between the latest waveform for 0.5 seconds and the waveform for the past 1.65 seconds of each of the heartbeat waveform HW1 to the heartbeat waveform HW4. To be specific, the calculation is performed, for example, using an array AR located in the storage unit 35. Here, calculation for the heartbeat waveform HW1 will be described as an example. Calculations for the heartbeat waveform HW2 to the heartbeat waveform HW4 are similar.

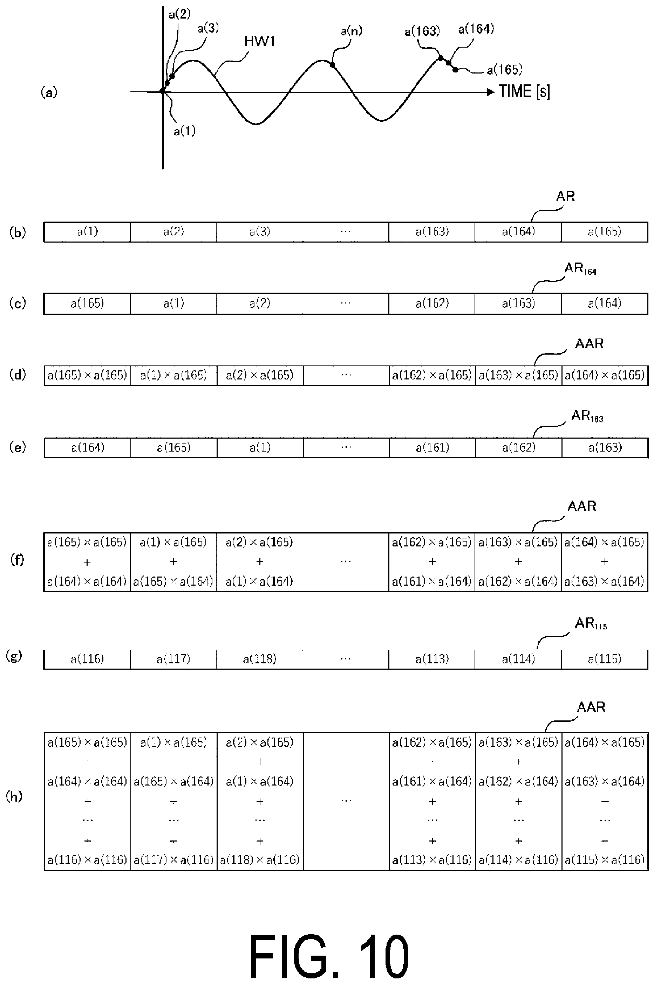

The array AR (FIG. 10(b)) can store 165 elements. The array AR stores sampled values a(n) (n = 1, 2, ⋯ 165) of the amplitude AM of the heartbeat waveform HW1 every 0.01 seconds (i.e., sampling period after downsampling in the waveform acquisition step S2). Each of the sampled values stored in the array AR corresponds to the heartbeat waveform HW1 for 1.65 seconds (FIG. 10(a)).

During a period when the body movement determination flag is set by the body movement determination unit 32, zero is stored as the sampled value a(n) of the amplitude AR.

First, the heart rate calculation unit 34 creates an array $AR_{164}$ by shifting the array AR - 164 times (FIG. 10(c)). Then, each element of the array $AR_{164}$ is multiplied by the sampled value a(165), and a multiplication result is stored in a multiplication array AAR (FIG. 10(d)). At this time, the data stored in the multiplication array AAR indicates the autocorrelation function of the heartbeat waveform HW1 calculated using a calculation window of 1.65 seconds, for the latest 0.01 seconds.

Subsequently, the heart rate calculation unit 34 creates an array $AR_{163}$ by shifting the array AR -163 times (FIG. 10(e)). Then, each element of the array $AR_{163}$ is multiplied by the sampled value a(164), and a multiplication result is added to the multiplication array AAR (FIG. 10(f)). At this time, the data stored in the multiplication array AAR indicates the autocorrelation function of the heartbeat waveform HW1 calculated using a calculation window of 1.65 seconds, for the latest 0.02 seconds.

Similarly, the heart rate calculation unit 34 creates an array $AR_N$ by shifting the array AR -N times (N = 162, 161, ⋯, 115). Then, each element of the array $AR_N$ is multiplied by the value of the sampled value a(N + 1), and a multiplication result is added to the multiplication array AAR. The heart rate calculation unit 34 repeats these procedures, and at a 50th time, creates an array $AR_{115}$ by shifting the array AR -115 times (FIG. 10(g)). Then, each element of the array $AR_{115}$ is multiplied by the value of a(116), and a multiplication result is added to the multiplication array AAR (FIG. 10(h)). At this time, the data stored in the multiplication array AAR indicates the autocorrelation function of the heartbeat waveform HW1 calculated using a calculation window of 1.65 seconds, for the latest 0.5 seconds.

In step S52, the heart rate calculation unit 34 determines whether the multiplication results for 50 times (0.5 seconds) have been added to the multiplication array AAR. Then, if the multiplication results for 50 times (0.5 seconds) have not been added, the heart rate calculation unit 34 returns to S51, and if the multiplication results for 50 times (0.5 seconds) have been added, the calculation result stored in the multiplication array AAR is stored at the storage unit 35 (S53).

The heart rate calculation unit 34 executes step S52 and step S53 for each of the heartbeat waveform HW1 to the heartbeat waveform HW4. Hereinafter, the autocorrelation functions calculated using a calculation window of 1.65 seconds for the heartbeat waveforms HW1, HW2, HW3, and HW4 for 0.5 seconds stored in step S53 are referred to as partial autocorrelation functions PAF1, PAF2, PAF3, and PAF4, respectively.

The heart rate calculation unit 34 stores the partial autocorrelation functions PAF1 to PAF4 in the storage unit 35 using a FIFO method. The storage unit 35 has an area for storing 16 each of the autocorrelation functions PAF1 to PAF4.

In step S54, the heart rate calculation unit 34 adds up the 16 autocorrelation functions PAF1, PAF2, PAF3, and PAF4 stored at the storage unit 35, respectively, to calculate the autocorrelation functions AF1, AF2, AF3, and AF4. The heart rate calculation unit 34 stores the calculated autocorrelation function AF1 to autocorrelation function AF4 at the storage unit 35. The autocorrelation function AF1 to the autocorrelation function AF4 indicate autocorrelation functions of the heartbeat waveform HW1 to the heartbeat waveform HW4, respectively, calculated using a calculation window of 1.65 seconds for the most recent 8 seconds.

In step S55, the heart rate calculation unit 34 adds up the autocorrelation function AF1 to the autocorrelation function AF4 stored at the storage unit 35 to calculate a total autocorrelation function AF. The heart rate calculation unit 34 stores the calculated total autocorrelation function AF at the storage unit 35.

In step S56, the heart rate calculation unit 34 determines whether the body movement occurrence period in the most recent eight seconds (hereinafter appropriately referred to as "calculation target time") related to the calculation of the autocorrelation functions AF1 to AF4 and the total autocorrelation function AF is shorter than one third of the whole period (eight seconds).

If the heart rate calculation unit 34 determines that the body movement occurrence period is equal to or longer than one third of the whole calculation target period (S56: NO), the heart rate calculation unit 34 outputs an error (S58). During a period when body movement occurs at the subject S, the heartbeat waveform is disturbed by the influence of the body movement, making it difficult to determine the cardiac cycle and calculate the heart rate with high accuracy.

If the heart rate calculation unit 34 determines that the body movement occurrence period is shorter than one third of the whole calculation target period (S56: YES), the heart rate calculation unit 34 calculates the heart rate of the subject S in step S57. The heart rate calculation unit 34 detects the peaks of the autocorrelation functions AF1 to AF4 and the total autocorrelation function AF using at least one of the following (i) to (iv) based on the autocorrelation functions AF1 to AF4 and the total autocorrelation function AF.

(i) Among the heartbeat waveform HW1 to the heartbeat waveform HW4, the waveform having the greatest power of the signal amplitude during the calculation target period is determined, and the autocorrelation function corresponding to the determined waveform is used. In this case, for example, when it is determined that the power of the signal amplitude of the heartbeat waveform HW1 is the largest, the autocorrelation function AF1 is used.

(ii) Among the autocorrelation function AF1 to the autocorrelation function AF4, the autocorrelation function having the largest absolute peak value is used.

(iii) The autocorrelation function AF1 is normalized by the power of the signal amplitude of the heartbeat waveform HW1, and similarly, the autocorrelation function AF2 to the autocorrelation function AF4 are normalized by the power of the signal amplitudes of the heartbeat waveform HW2 to the heartbeat waveform HW4, respectively. Then, among the normalized autocorrelation functions AF1 to AF4, the normalized autocorrelation function having the largest absolute peak value is used.

(iv) The total autocorrelation function AF is used. Thus, the signal-to-noise ratio is improved and the accuracy of the heart rate calculation is increased.

To be specific, the heart rate calculation unit 34 calculates the heart rate of the subject S, for example, through the following steps. Here, calculation using the autocorrelation function AF1 will be described. A calculation method described below can be similarly applied to any of (i) to (iv) described above.

The heart rate calculation unit 34 first executes peak detection on the autocorrelation function AF1. At this time, the heart rate calculation unit 34 determines a range where the peak detection is executed, taking into consideration the estimated value of the cardiac cycle of the subject S estimated in the cardiac cycle estimation step S4.

As described above, it is considered that the peak of the autocorrelation function AF1 appears at a position corresponding to the period of the heartbeat waveform HW1. Therefore, the heart rate calculation unit 34 takes into consideration the estimated value of the cardiac cycle (i.e., the period of the heartbeat waveform HW1) of the subject S estimated in the cardiac cycle estimation step S4 and limits the range where peak detection is executed to around the estimated value. Thus, it is possible to increase the reliability of the detection result while reducing the load of calculation processing in peak detection.

Subsequently, the heart rate calculation unit 34 performs an interpolation of the autocorrelation function AF1 before and after the detected peak.

The autocorrelation function AF1 to the autocorrelation function AF4 and the total autocorrelation function AF are all based on at least one of the heartbeat waveform HW1 to the heartbeat waveform H4, represented by the sampling period of 0.01 seconds. Therefore, data points for the autocorrelation function AF1 to the autocorrelation function AF4 and the total autocorrelation function AF are also obtained at 0.01 seconds gaps.

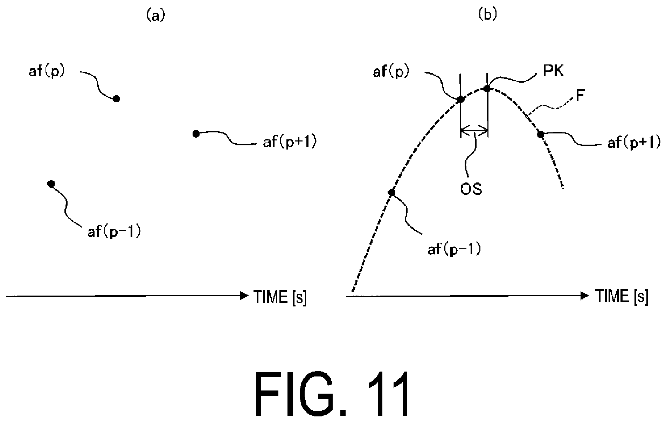

Here, it is assumed that as a result of peak detection for the autocorrelation function AF1, a data point of an amplitude af(p) shown in FIG. 11(a) is detected as a peak. At this time, an amplitude af(p - 1) at a data point one before the amplitude af(p) and an amplitude af(p + 1) at a data point one after the amplitude af(p) both indicate values smaller than the amplitude af(p), so when looking at only these three data points, the amplitude af(p) appears to correspond to the peak. However, since the data points of the autocorrelation function AF1 are obtained at 0.01 second gaps, a true peak of the autocorrelation function AF1 may exist between the amplitude af(p) and the amplitude af(p - 1) or between the amplitude af(p) and the amplitude af(p + 1).

Therefore, the heart rate calculation unit 34 performs an interpolation on the autocorrelation function AF1 to find a true peak PK of the autocorrelation function AF1. To be specific, for example, the interpolation is performed by fitting a negative quadratic function F (FIG. 11(b)) to a curve of the autocorrelation function AF1 in the vicinity of the peak detected by the peak detection. Thus, the following equation indicating a deviation OS between the peak detected by the peak detection and the true peak PK is obtained.

[Mathematical Expression 1]

Equation 1:

$$OS = 0.5 \times \frac{\{af(p-1) - af(p+1)\}}{\{af(p+1) + af(p-1) - 2 \times af(p)\}} \times Ts$$

Here, Ts is the sampling period of the data points of the autocorrelation function AF1 (0.01 [s] in the present embodiment).

FIG. 11(b) shows a state where the quadratic function F is fitted by the interpolation. The heart rate calculation unit 34 detects the peak PK of the quadratic function F as the true peak.

Subsequently, the heart rate calculation unit 34 determines a lag time L corresponding to the true peak (i.e., the peak PK of the quadratic function F) to be a cardiac cycle T of the subject S. Then, the heart rate calculation unit 34 calculates a heart rate HR [bpm] of the subject S using the following (Equation 2).

[Mathematical Expression 2]

Equation 2

$$HR = 60/T \text{ [bpm]}$$

The heart rate calculation unit 34 sequentially calculates the heart rate HR based on at least one of the autocorrelation functions AF1 to AF4 and the total autocorrelation function AF. The autocorrelation functions AF1 to AF4 and the total autocorrelation function AF are calculated sequentially. The heart rate calculation unit 34 calculates the heart rate HR, for example, once every 0.01 seconds.

Display Step S6

In the display step S6, the display control unit 41 of the user terminal 40 sequentially acquires the heart rate HR sequentially calculated by the heart rate calculation unit 34 from the biological information acquisition unit 30 and displays the heart rate HR at the display unit 42. Further, in the display step S6, in addition to or in lieu of the display using the display unit 42, a notification may be made using the notification unit 43. In this case, for example, the display control unit 41 emits a notification sound when the heart rate HR of the subject S deviates from a specified range and notifies the user of the biological information acquisition system 100, such as a doctor, a nurse, or a caregiver, of an abnormal heartbeat state.

To be specific, the display control unit 41 performs display in the following manner, for example.

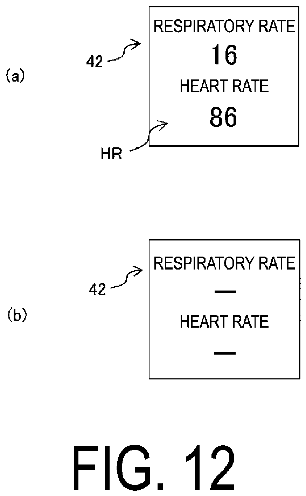

In the present embodiment, the display control unit 41 sequentially acquires the heart rate HR calculated by the heart rate calculation unit 34, stores the heart rate HR at the storage unit 45, and displays a median of multiple heart rates HR for the most recent minute at the display unit 42 (FIG. 12(a)). Thus, effects of momentary fluctuations in the heart rate HR due to disturbances or the like can be suppressed. This point also leads to suppression of false notification in a case where audio notification is made by the notification unit 43 when the heart rate HR exceeds the specified threshold.

The display control unit 30 does not display the heart rate HR at the display unit 42 during a period when the subject S is not present on the bed BD (FIG. 12(b)). The period when the subject S is not present on the bed BD may specifically be, for example, a period when the biological information acquisition unit 30 (respiratory rate calculation unit, weight calculation unit) does not calculate the respiratory rate of the subject S, a period when the weight of the subject S detected by the load detection unit 1 is equal to or less than a specified value, or the like.

To be specific, the biological information acquisition unit 30 can, for example, sequentially calculate the center of gravity position of the subject S based on the load signals $s_1$ to $s_4$, and calculate the respiratory rate of the subject S based on a temporal fluctuation of the center of gravity position of the subject S in response to the respiration of the subject S. Alternatively, the biological information acquisition unit 30 may perform Fourier analysis on at least one of the load signals $s_1$ to $s_4$, identify a peak frequency appearing at a frequency band corresponding to respiration, and calculate the respiratory rate of the subject S by regarding the identified peak frequency as the frequency of the respiration of the subject S.

The biological information acquisition unit 30 can calculate the weight of the subject S by adding up the load

signals $s_1$ to $s_4$.

The biological information acquisition unit 30 may calculate the heart rate based on disturbances (e.g., air vibrations caused by operation of surrounding equipment such as an air conditioner), even during periods when the subject S is not present on the bed BD or the like. Therefore, by stopping the display of the heart rate HR at the display unit 42 during a specified period when it can be assumed that the subject S is not present on the bed BD, it is possible to prevent erroneous display of the heart rate HR.

The effects of the biological information acquisition system 100 according to the present embodiment are summarized below.

The biological information acquisition system 100 according to the present embodiment first estimates the cardiac cycle of the subject S based on the heartbeat waveform HW1 or the like in the cardiac cycle estimation step S4. Then, peak detection of the autocorrelation function AF1 or the like is performed within a range matching the estimated cardiac cycle, and the heart rate HR of the subject S is calculated based on the detected peak. Thus, the biological information acquisition system 100 according to the present embodiment performs peak detection of the autocorrelation function AF1 or the like, taking into consideration the previously estimated cardiac cycle. Therefore, even when the heartbeat waveform HW1 or the like is distorted and the periodicity is disturbed, it is possible to determine the peak position of the autocorrelation function AF1 or the like with high accuracy and calculate the heart rate HR with high accuracy. In addition, the calculation processing load for peak detection is small.

In the heart rate calculation step S5, the biological information acquisition system 100 according to the present embodiment does not calculate the entire autocorrelation function for the target period at once but divides the entire autocorrelation function into partial autocorrelation functions, calculates the partial autocorrelation functions, and adds up the calculated partial autocorrelation functions. Therefore, the autocorrelation function at each timing can be calculated with a smaller amount of calculation. Thus, a small calculation processing load is particularly advantageous when implementing the biological information acquisition system 100 as an embedded program in a microcomputer.

In the heart rate calculation step S5, the biological information acquisition system 100 according to the present embodiment detects a true peak by performing an interpolation and calculates the heart rate HR of the subject S based on the true peak. Therefore, the heart rate HR of the subject S can be determined with higher accuracy.

In the display step S6, the biological information acquisition system 100 according to the present embodiment stops display of the heart rate HR at the display unit 42 during a specified period when it can be estimated that the subject S is not present on the bed BD. Therefore, the biological information acquisition system 100 according to the present embodiment can prevent erroneous display of the heart rate HR.

Modifications

In the biological information acquisition system 100 of the above embodiment, the following modifications may also be employed.

In the cardiac cycle estimation step S4 of the above embodiment, each time the cardiac cycle estimation unit 33 identifies the falling zero crossing points fx, the cardiac cycle estimation unit 33 determines whether the gap between the falling zero crossing points fx, the peak p, the bottom b, and the like in the immediately preceding period satisfy the specified conditions. However, no such limitation is intended. A similar determination may be made each time the rising zero crossing point rx is identified.

In the cardiac cycle estimation step S4 of the above embodiment, the determination of whether the amplitude AM (positive value) at the peak p is smaller than the body movement threshold THp and/or the determination of whether the amplitude AM (negative value) at the bottom b is greater than the body movement threshold THn may be omitted. In the cardiac cycle estimation step S4 of the above embodiment, the determination of whether the fluctuation ratio of the gap PR of the immediately preceding period to the gap PR of the period one period before this immediately preceding period is within a specified range may be omitted.

In the cardiac cycle estimation step S4 of the above embodiment, the median of the gaps PR corresponding to the eight periods stored at the storage unit 35 is set as the estimated value of the cardiac cycle of the subject S, but the number of gaps PR is not limited to eight. The number of gaps PR stored in the storage unit 35 may be any number greater than one or may be one. The cardiac cycle estimation unit 33 may use a median or an average of any number of gaps PR greater than one as the estimated value of the cardiac cycle of the subject S.

In the cardiac cycle estimation step S4 of the above embodiment, the cardiac cycle estimation unit 33 estimates the cardiac cycle of the subject S using the heartbeat waveform HW1, but the heartbeat waveform is not limited to the heartbeat waveform HW1. The cardiac cycle estimation unit 33 can estimate the cardiac cycle of the subject S using at least one of the heartbeat waveforms HW1 to HW4.

In the heart rate calculation step S5 of the above embodiment, the heart rate calculation unit 34 sequentially calculates the partial autocorrelation functions PAF1 to PAF4 between the respiratory waveforms HW1 to HW4 for the latest 0.5 seconds and the respiratory waveforms HW1 to HW4 for the past 1.65 seconds, and adds up the respective partial autocorrelation functions PAF1 to PAF4 for 8 seconds to calculate the autocorrelation functions AF1 to AF4. However, the calculation target periods are not limited to 0.5 seconds or 8 seconds.

The calculation target period of the partial autocorrelation functions PAF1 to PAF4 is not limited to 0.5 seconds and can be any period. In addition, the calculation target period of the autocorrelation functions AF1 to AF4 is not limited to 8 seconds and can be any period.

That is, the heart rate calculation unit 34 may be in any aspect including calculating partial autocorrelation functions PAF1 to PAF4 (first autocorrelation functions) of the heartbeat waveforms HW1 to HW4 included in a specified period (first period), calculating partial autocorrelation functions PAF1 to PAF4 (second autocorrelation functions) of the heartbeat waveforms HW1 to HW4 included in a specified period (second period) after the specified period, calculating partial autocorrelation functions PAF1 to PAF4 (third autocorrelation functions) of the heartbeat waveforms HW1 to HW4 included in a specified period (third period) after the specified period, calculating a sum of the first autocorrelation function and the second autocorrelation function as the autocorrelation functions AF1 to AF4 at a specified timing (first time), and calculating a sum of the second autocorrelation function and the third autocorrelation function as the autocorrelation functions AF1 to AF4 at a subsequent specified timing (second time).

In the heart rate calculation step S5 of the above embodiment, the heart rate calculation unit 34 may calculate the autocorrelation functions AF1 to AF4 each time, without calculating the partial autocorrelation functions PAF1 to PAF4. In this case, for example, the autocorrelation functions of the heartbeat waveform HW1 to the heartbeat waveform HW4 calculated using a calculation window of 1.65 seconds for the latest 8 seconds are calculated every 0.5 seconds.

In the heart rate calculation step S5 of the above embodiment, if the heart rate calculation unit 34 determines that the body movement occurrence period is equal to or longer than one third of the whole calculation target period (S56: YES), the heart rate calculation unit 34 outputs an error (S58). However, outputting an error is not limited to the case where the body movement occurrence period is equal to or longer than one third of the whole calculation target period, and the heart rate calculation unit 34 may output an error in step S56 when the body movement occurrence period is any ratio (1/2, 1/4, etc.) to the calculation target period. Alternatively, the heart rate calculation unit 34 may output an error when at least part of the calculation target period is the body movement occurrence period.

In the heart rate calculation step S5 of the above embodiment, the heart rate calculation unit 34 does not need to perform the interpolation. In this case, the peak of the autocorrelation function detected by the peak detection is used to determine the cardiac cycle T of the subject S and calculate the heart rate HR [bpm] of the subject S.

In the above embodiment, the display control unit 30 does not display the heart rate HR at the display unit 42 during a period when the subject S is not present on the bed BD. However, no such limitation is intended. The display control unit 30 can set the display of the heart rate HR at the display unit 42 to any different display modes between during a period when the subject S is present on the bed BD and during a period when the subject S is not present on the bed BD.

To be specific, for example, the display control unit 30 may provide normal display during a period when the subject S is present on the bed BD, and may provide display in red characters, flashing display, or the like during a period when the subject S is not present on the bed BD. Note that in this specification and the present invention, "non-display" is also one mode of the display method. Thus, by making the display mode of the heart rate HR when the subject S is not present on the bed different from the display mode of the heart rate HR when the subject S is present on the bed, the user or the like can intuitively understand that the displayed heart rate is not the heart rate of the subject S.

The biological information acquisition unit 30 of the above embodiment does not need to include the body movement determination unit 32. In this case, the processing related to the body movement determination in the above embodiment does not need to be executed.

In the heartbeat information acquisition system 100 of the above embodiment, the heart rate calculation unit 34 calculates the heart rate based on the calculation of the autocorrelation value. However, the calculation of the heart rate is not limited to being based on the calculation of the autocorrelation value. Various methods may be used to calculate the heart rate based on the heartbeat waveform.

To be specific, for example, the heart rate calculation unit 34 may perform Fourier analysis on at least one of the load signals $s_1$ to $s_4$, identify a peak frequency appearing at a frequency band corresponding to a heartbeat, and calculate the respiratory rate of the subject S by regarding the identified peak frequency as the frequency of the heartbeat of the subject S.

In this aspect, when identifying the peak frequency, the estimated value of the cardiac cycle of the subject S estimated in the cardiac cycle estimation step S4 may be taken into consideration.

In the present specification and the present invention, a negative amplitude value being smaller than the threshold (negative value) means that the absolute value of the negative amplitude value is greater than the absolute value of the threshold (negative value). The comparison of the amplitude value (negative value) and the threshold (negative value) defined in the present invention includes an aspect for comparing the absolute value of the amplitude value (negative value) with the absolute value of the threshold (negative value).

In the biological information acquisition system 100 of the above embodiment, one biological information acquisition unit 30 is connected to the user terminal 40. However, the number of biometric information acquisition units 30 connected to the user terminal 40 is not limited to one. Multiple biological information acquisition units 30 may be connected to the user terminal 40. Alternatively, the biological information acquisition unit 30 and the user terminal 40 may be integrated, and the user terminal 40 may be regarded as part of the heart rate acquisition unit of the present invention.

The biological information acquisition system 100 of the above embodiment does not necessarily need to include all of the load detectors 11 to 14 but may only include at least one of the load detectors 11 to 14. The load detectors do not necessarily need to be positioned at the four corners of the bed and can be disposed at any position so that the load of the subject on the bed and the fluctuation of the load can be detected. As the load detectors 11 to 14, for example, a force sensor can be used without being limited to a load sensor using a beam-type load cell.

In the biological information acquisition system 100 of the above embodiment, the load detection unit 1 may be multiple pressure-sensitive sensors (pressure sensors) arranged in a matrix under a sheet.

The load detectors 11 to 14 may be combined with the bed BD integrally or detachably, and a bed system BDS (FIG. 2) consisting of the bed BD and the biological information acquisition system 100 according to the present embodiment can be configured.

The display control unit 41 and the display unit 42 of the above embodiment may be combined with any heart rate calculation unit to configure a heart rate calculation device. Such a heart rate calculation device can more appropriately display the acquired heart rate.

In the biological information acquisition device 30 of the above embodiment, the cardiac cycle estimation unit 33 may be omitted. Also in this case, the heart rate can be obtained more efficiently by the heart rate calculation unit 34.

As long as the features of the present invention are maintained, the present invention is not limited to the embodiment described above, and other forms considered within the scope of the technical concept of the present invention are also included within the scope of the present invention.

Reference Signs List

1 Load detection unit; 11, 12, 13, 14 Load detector; 3 Biological information acquisition unit; 31 Heartbeat waveform acquisition unit; 32 Body movement determination unit; 33 Cardiac cycle estimation unit; 34 Heart rate calculation unit; 35 Storage unit; 40 User terminal; 41 Display control unit; 42 Display unit; 43 Notification unit; 44 Input unit; 45 Storage unit; BD Bed; BDS Bed system

**Claims**

1. A heart rate acquisition device configured to acquire a heart rate of a subject on a bed, the heart rate acquisition device comprising:

   a heartbeat waveform acquisition unit configured to acquire a heartbeat waveform indicating a temporal fluctuation of a load value corresponding to a heartbeat of the subject based on an output of a load detector located at the bed;
   a cardiac cycle estimation unit configured to obtain an estimated value of a cardiac cycle of the subject based on transitions of the heartbeat waveform; and
   a heart rate calculation unit configured to calculate a heart rate of the subject based on the heartbeat waveform and the estimated value.

2. The heart rate acquisition device according to claim 1, wherein the cardiac cycle estimation unit obtains the estimated value based on identification of a bottom of the heartbeat waveform, a rising zero crossing point of the heartbeat waveform, a peak of the heartbeat waveform, and a falling zero crossing point of the heartbeat waveform.

3. The heart rate acquisition device according to claim 2, wherein when a gap between two consecutive rising zero crossing points or two consecutive falling zero crossing points is within a specified range, an amplitude of the heartbeat waveform at the bottom between the two consecutive rising zero crossing points or the two consecutive falling zero crossing points is smaller than a bottom amplitude threshold, and an amplitude of the heartbeat waveform at the peak between the two consecutive rising zero crossing points or the two consecutive falling zero crossing points is greater than a peak amplitude threshold, the cardiac cycle estimation unit sets the gap between the two consecutive rising zero crossing points or the two consecutive falling zero crossing points as the estimated value.

4. The heart rate acquisition device according to claim 3, wherein when the amplitude of the heartbeat waveform at the bottom between the two consecutive rising zero crossing points or the two consecutive falling zero crossing points is greater than a bottom body movement threshold, and the amplitude of the heartbeat waveform at the peak between the two consecutive rising zero crossing points or the two consecutive falling zero crossing points is smaller than a peak body movement threshold, the cardiac cycle estimation unit sets the gap between the two consecutive rising zero crossing points or the two consecutive falling zero crossing points as the estimated value.

5. The heart rate acquisition device according to claim 3 or 4, wherein when the gap between the two consecutive rising zero crossing points or the two consecutive falling zero crossing points is equal to or greater than a specified value, the cardiac cycle estimation unit determines that the gap between the two consecutive rising zero crossing points or the two consecutive falling zero crossing points is within the specified range.

6. The heart rate acquisition device according to any one of claims 3 to 5, wherein the cardiac cycle estimation unit sequentially identifies the rising zero crossing point or the falling zero crossing point and calculates a gap between the two consecutive rising zero crossing points or the two consecutive falling zero crossing points, and when a fluctuation ratio of the gap calculated at a certain timing to the gap calculated immediately before the certain timing is within a specified range, the cardiac cycle estimation unit determines that the gap is within the specified range.

7. The heart rate acquisition device according to any one of claims 1 to 6, wherein
calculation of the heart rate at the heart rate calculation unit includes:

calculating an autocorrelation function of the heartbeat waveform,
detecting a peak of the autocorrelation function, and
calculating the heart rate based on a position of the peak detected, and
the heart rate calculation unit determines a range where detection of the peak is executed based on the estimated value.

8. The heart rate acquisition device according to claim 7, wherein
in the calculation of the heart rate at the heart rate calculation unit, the calculation of the heart rate based on a position of the peak detected includes:

performing an interpolation around the peak detected, and
calculating the heart rate based on a position of a peak identified by the interpolation.

9. The heart rate acquisition device according to claim 7 or 8, wherein
calculation of the autocorrelation function at the heart rate calculation unit includes:

calculating a first autocorrelation function of the heartbeat waveform included in a first period,
calculating a second autocorrelation function of the heartbeat waveform included in a second period after the first period,
calculating a third autocorrelation function of the heartbeat waveform included in a third period after the second period,
calculating a sum of the first autocorrelation function and the second autocorrelation function at a first time as the autocorrelation function, and
calculating a sum of the second autocorrelation function and the third autocorrelation function at a second time after the first time as the autocorrelation function.

10. The heart rate acquisition device according to any one of claims 7 to 9, wherein

the load detector includes a first load detector and a second load detector,
the heartbeat waveform acquisition unit acquires a first heartbeat waveform based on an output of the first load detector and a second heartbeat waveform based on an output of the second load detector, and
the autocorrelation function calculated by the heart rate calculation unit is a sum of an autocorrelation function of the first heartbeat waveform and an autocorrelation function of the second heartbeat waveform.

11. The heart rate acquisition device according to any one of claims 7 to 10, further comprising:

a body movement determination unit configured to determine presence or absence of body movement of the subject based on a comparison between an amplitude of the heartbeat waveform and a body movement threshold, wherein
in a case where a ratio of a period when the body movement occurs at the subject in a calculation target period of the autocorrelation function is equal to or greater than a threshold, the heart rate calculation unit does not calculate the heart rate based on the autocorrelation function.

12. The heart rate acquisition device according to any one of claims 1 to 11, further comprising:

a display unit configured to display the heart rate calculated by the heart rate calculation unit; and
a display control unit configured to control display content of the display unit, wherein
the display control unit changes a display mode of the heart rate calculated by the heart rate calculation unit at the display unit between a period when the subject is present on the bed and a period when the subject is not present on the bed.

13. The heart rate acquisition device according to claim 12, further comprising:

a weight calculation unit configured to calculate a weight of the subject based on the output of the load detector and/or a respiratory rate calculation unit configured to calculate a respiratory rate of the subject based on the output of the load detector, wherein
when the weight of the subject calculated is equal to or less than a threshold and/or when the respiratory rate of the subject is not calculated, the display control unit determines that the subject is not present on the bed.

14. The heart rate acquisition device according to claim 12 or 13, wherein

the heart rate calculation unit sequentially calculates the heart rate, and
the display control unit displays a median of a plurality of the heart rates sequentially calculated at the display unit.

15. A heart rate acquisition device configured to acquire a heart rate of a subject on a bed, the heart rate acquisition device comprising:

a heart rate calculation unit configured to calculate a heart rate of the subject based on a temporal fluctuation of an output of a load detector located at the bed corresponding to a heartbeat of the subject;
a display unit configured to display the heart rate calculated by the heart rate calculation unit; and
a display control unit configured to control display content of the display unit, wherein
the display control unit changes a display mode of the heart rate calculated by the heart rate calculation unit at the display unit between a period when the subject is present on the bed and a period when the subject is not present on the bed.

16. The heart rate acquisition device according to claim 15, further comprising:

a weight calculation unit configured to calculate a weight of the subject based on the output of the load detector and/or a respiratory rate calculation unit configured to calculate a respiratory rate of the subject based on the output of the load detector, wherein
when the weight of the subject calculated is equal to or less than a threshold and/or when the respiratory rate of the subject is not calculated, the display control unit determines that the subject is not present on the bed.

17. The heart rate acquisition device according to claim 15 or 16, wherein

the heart rate calculation unit sequentially calculates the heart rate, and
the display control unit displays a median of a plurality of the heart rates sequentially calculated at the display unit.

18. A heart rate acquisition device configured to acquire a heart rate of a subject on a bed, the heart rate acquisition device comprising:

a heartbeat waveform acquisition unit configured to acquire a heartbeat waveform indicating a temporal fluctuation of a load value corresponding to a heartbeat of the subject based on an output of a load detector located at the bed; and
a heart rate calculation unit configured to calculate a heart rate of the subject based on the heartbeat waveform, wherein
calculation of the heart rate at the heart rate calculation unit includes:

calculating an autocorrelation function of the heartbeat waveform,

detecting a peak of the autocorrelation function, and

calculating the heart rate based on a position of the peak detected, and

calculation of the autocorrelation function includes:

calculating a first autocorrelation function of the heartbeat waveform included in a first period,

calculating a second autocorrelation function of the heartbeat waveform included in a second period after the first period,

calculating a third autocorrelation function of the heartbeat waveform included in a third period after the second period,

calculating a sum of the first autocorrelation function and the second autocorrelation function at a first time as the autocorrelation function, and

calculating a sum of the second autocorrelation function and the third autocorrelation function at a second time after the first time as the autocorrelation function.

19. The heart rate acquisition device according to claim 18, wherein

in the calculation of the heart rate at the heart rate calculation unit, the calculation of the heart rate based on a position of the peak detected includes:

performing an interpolation around the peak detected, and

calculating the heart rate based on a position of a peak identified by the interpolation.

20. A bed system comprising:

a bed;

a load detector located at the bed; and

the heart rate acquisition device according to any one of claims 1 to 19.

FIG. 1

# FIG. 2

| LOAD DETECTION STEP | S1 |

↓

| HEARTBEAT WAVEFORM ACQUISITION STEP | S2 |

↓

| BODY MOVEMENT DETERMINATION STEP | S3 |

↓

| CARDIAC CYCLE ESTIMATION STEP | S4 |

↓

| HEART RATE CALCULATION STEP | S5 |

↓

| DISPLAY STEP | S6 |

# FIG. 3

FIG. 4

START

WAVEFORM TRANSITION TRACKING STEP — S41

FALLING ZERO CROSSING POINT IDENTIFIED? — S42
NO →
YES

WAVEFORM OF IMMEDIATELY PRECEDING PERIOD SATISFY SPECIFIED CONDITIONS? — S43
NO →
YES

GAP OF IMMEDIATELY PRECEDING PERIOD SATISFY SPECIFIED CONDITIONS? — S44
NO →
YES

STORE GAP OF IMMEDIATELY PRECEDING PERIOD IN STORAGE UNIT — S45

STORE GAPS FOR EIGHT PERIODS — S46
NO →
YES

MEDIAN → ESTIMATED VALUE OF CARDIAC CYCLE — S47

END

# FIG. 5

FIG. 6

EP 4 603 008 A1

| STATE OF HEARTBEAT WAVEFORM HW1 | FALLING | BOTTOM b | RISING | RISING ZERO CROSSING POINT rx | RISING | PEAK p | FALLING | FALLING ZERO CROSSING POINT fx | FALLING |
|---|---|---|---|---|---|---|---|---|---|
| SIGN OF SLOPE OF HEARTBEAT WAVEFORM HW1 | NEGATIVE | NEGATIVE → POSITIVE | POSITIVE | POSITIVE | POSITIVE | POSITIVE → NEGATIVE | NEGATIVE | NEGATIVE | NEGATIVE |
| SIGN OF HEARTBEAT WAVEFORM HW1 | NEGATIVE | NEGATIVE | NEGATIVE | NEGATIVE → POSITIVE | POSITIVE | POSITIVE | POSITIVE | POSITIVE → NEGATIVE | NEGATIVE |
| STATE TRANSITION VARIABLE WS | 4 | 4 → 1 | 1 | 1 → 2 | 2 | 2 → 3 | 3 | 3 → 4 | 4 |

<div align="center">

# FIG. 7

</div>

EP 4 603 008 A1

W(t)

w(t)

(a)                                                    TIME [s]

W(t+0.5)

w(t+0.5)

(b)                                                    TIME [s]

W(t+1.0)

w(t)          w(t+1.0)

w(t+0.5)

(c)                                                    TIME [s]

# FIG. 8

START

CALCULATE AUTOCORRELATION FUNCTION — S51

AUTOCORRELATION FUNCTIONS FOR 0.5 SECONDS CALCULATED? — S52

NO

YES

AUTOCORRELATION FUNCTIONS FOR 0.5 SECONDS STORED IN STORAGE UNIT — S53

AUTOCORRELATION FUNCTIONS FOR EIGHT SECONDS ADDED UP — S54

CALCULATE TOTAL AUTOCORRELATION FUNCTION — S55

BODY MOVEMENT OCCURRENCE RATE LESS THAN 1/3? — S56

NO

YES

CALCULATE HEART RATE — S57

OUTPUT ERROR — S58

END

# FIG. 9

(a)

a(2) a(3) HW1 a(n) a(163) a(164)
a(165)
TIME [s]
a(1)

AR
(b)

| a(1) | a(2) | a(3) | ⋯ | a(163) | a(164) | a(165) |
|---|---|---|---|---|---|---|

$AR_{164}$
(c)

| a(165) | a(1) | a(2) | ⋯ | a(162) | a(163) | a(164) |
|---|---|---|---|---|---|---|

AAR
(d)

| a(165) × a(165) | a(1) × a(165) | a(2) × a(165) | ⋯ | a(162) × a(165) | a(163) × a(165) | a(164) × a(165) |
|---|---|---|---|---|---|---|

$AR_{163}$
(e)

| a(164) | a(165) | a(1) | ⋯ | a(161) | a(162) | a(163) |
|---|---|---|---|---|---|---|

AAR
(f)

| a(165) × a(165) + a(164) × a(164) | a(1) × a(165) + a(165) × a(164) | a(2) × a(165) + a(1) × a(164) | ⋯ | a(162) × a(165) + a(161) × a(164) | a(163) × a(165) + a(162) × a(164) | a(164) × a(165) + a(163) × a(164) |
|---|---|---|---|---|---|---|

$AR_{115}$
(g)

| a(116) | a(117) | a(118) | ⋯ | a(113) | a(114) | a(115) |
|---|---|---|---|---|---|---|

AAR
(h)

| a(165) × a(165) − a(164) × a(164) − ⋯ − a(116) × a(116) | a(1) × a(165) + a(165) × a(164) + ⋯ + a(117) × a(116) | a(2) × a(165) + a(1) × a(164) + ⋯ + a(118) × a(116) | ⋯ | a(162) × a(165) + a(161) × a(164) + ⋯ + a(113) × a(116) | a(163) × a(165) + a(162) × a(164) + ⋯ + a(114) × a(116) | a(164) × a(165) + a(163) × a(164) + ⋯ + a(115) × a(116) |
|---|---|---|---|---|---|---|

# FIG. 10

(a)

af(p)

af(p+1)

af(p-1)

→ TIME [s]

(b)

af(p)

PK

F

af(p+1)

OS

af(p-1)

→ TIME [s]

# FIG. 11

(a)

42

HR

RESPIRATORY RATE

16

HEART RATE

86

(b)

42

RESPIRATORY RATE

—

HEART RATE

—

# FIG. 12

## INTERNATIONAL SEARCH REPORT

| International application No. |
| --- |
| **PCT/JP2023/036666** |

| A. | CLASSIFICATION OF SUBJECT MATTER |
| --- | --- |

*A61B 5/0245*(2006.01)i; *A61B 5/11*(2006.01)i
FI: A61B5/0245 A; A61B5/0245 200; A61B5/11 100

According to International Patent Classification (IPC) or to both national classification and IPC

| B. | FIELDS SEARCHED |
| --- | --- |

Minimum documentation searched (classification system followed by classification symbols)

A61B5/02-5/03; A61B5/06-5/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2022-72436 A (MINEBEAMITSUMI INC.) 17 May 2022 (2022-05-17) paragraphs [0020]-[0110], fig. 1-11 | 1-8, 10-17, 20 |
| A | | 9, 18-19 |
| Y | JP 63-277034 A (TOITU CO., LTD.) 15 November 1988 (1988-11-15) p. 3, upper right column, line 5 to p. 4, upper right column, line 13, claim 1, fig. 1-5 | 1-8,10-14,20 |
| Y | JP 2016-149 A (OMRON HEALTHCARE CO., LTD.) 07 January 2016 (2016-01-07) paragraphs [0129]-[0133] | 2-6 |
| Y | JP 2019-474 A (GLORY LTD.) 10 January 2019 (2019-01-10) paragraphs [0091], [0101], [0102], claim 5 | 2-6 |
| Y | JP 2020-174741 A (MINEBEAMITSUMI INC.) 29 October 2020 (2020-10-29) paragraphs [0054]-[0057], [0074], [0087], [0095] | 2-6,13,16 |
| Y | JP 2009-501557 A (EARLYSENS LTD.) 22 January 2009 (2009-01-22) paragraphs [0234]-[0237] | 2-6 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **15 December 2023** | **09 January 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2023/036666** |

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2002-34941 A (SEIKO PRECISION INC.) 05 February 2002 (2002-02-05)<br>paragraphs [0015], [0022], [0026]-[0030] | 3-6 |
| Y | JP 2006-122377 A (SANYO ELECTRIC CO., LTD.) 18 May 2006 (2006-05-18)<br>paragraphs [0005]-[0007] | 3-6 |
| Y | JP 3-159634 A (SANYO ELECTRIC CO., LTD.) 09 July 1991 (1991-07-09)<br>p. 2, lower right column, lines 2-9, claim 2 | 3-6 |
| Y | JP 2010-119585 A (FUJITSU LTD.) 03 June 2010 (2010-06-03)<br>paragraph [0019] | 3-6 |
| Y | JP 8-322804 A (MATSUSHITA ELECTRIC INDUSTRIAL CO., LTD.) 10 December 1996<br>(1996-12-10)<br>paragraphs [0028], [0047] | 12-17, 20 |
| Y | JP 10-137206 A (SEIKO EPSON CORP.) 26 May 1998 (1998-05-26)<br>paragraph [0018] | 12-17, 20 |
| Y | JP 2016-123811 A (AISIN SEIKI CO., LTD.) 11 July 2016 (2016-07-11)<br>paragraphs [0013], [0014], [0016] | 13, 16 |
| Y | JP 62-295646 A (MATSUSHITA ELECTRIC WORKS, LTD.) 23 December 1987<br>(1987-12-23)<br>p. 2, lower right column, lines 10-19, p. 3, lower right column, lines 9-14 | 14, 17 |

Form PCT/ISA/210 (second sheet) (January 2015)

## INTERNATIONAL SEARCH REPORT
### Information on patent family members

| International application No. |
| --- |
| **PCT/JP2023/036666** |

| Patent document cited in search report | | | Publication date (day/month/year) | Patent family member(s) | | | Publication date (day/month/year) |
| --- | --- | --- | --- | --- | --- | --- | --- |
| JP | 2022-72436 | A | 17 May 2022 | WO | 2022/091933 | A1 | |
| JP | 63-277034 | A | 15 November 1988 | (Family: none) | | | |
| JP | 2016-149 | A | 07 January 2016 | (Family: none) | | | |
| JP | 2019-474 | A | 10 January 2019 | (Family: none) | | | |
| JP | 2020-174741 | A | 29 October 2020 | US 2022/0142582 A1 paragraphs [0063]-[0066], [0083], [0096], [0104] WO 2020/213431 A1 EP 3957963 A1 CN 113692523 A | | | |
| JP | 2009-501557 | A | 22 January 2009 | US 2006/0241510 A1 paragraphs [0587]-[0590] WO 2006/137067 A2 CN 101365373 A KR 10-2008-0034437 A | | | |
| JP | 2002-34941 | A | 05 February 2002 | (Family: none) | | | |
| JP | 2006-122377 | A | 18 May 2006 | (Family: none) | | | |
| JP | 3-159634 | A | 09 July 1991 | (Family: none) | | | |
| JP | 2010-119585 | A | 03 June 2010 | (Family: none) | | | |
| JP | 8-322804 | A | 10 December 1996 | (Family: none) | | | |
| JP | 10-137206 | A | 26 May 1998 | US 6036653 A column 6, lines 1-8 | | | |
| JP | 2016-123811 | A | 11 July 2016 | (Family: none) | | | |
| JP | 62-295646 | A | 23 December 1987 | (Family: none) | | | |

Form PCT/ISA/210 (patent family annex) (January 2015)

## EP 4 603 008 A1

**Patent documents cited in the description**

- JP 2022072436 A